# EUROPEAN PATENT APPLICATION

(11) **EP 4 623 865 A1**
(43) Date of publication of application: **01.10.2025**
(21) Application number: 24834584.5
(22) Date of filing: 26.02.2024
(51) Int. Cl.: A61F 2/16, A61F 9/007

(54) **PRE-LOADED INTRAOCULAR LENS IMPLANTATION SYSTEM**

(30) Priority: 06.02.2024 CN 202420287766 U
(71) Applicant: Wuxi Vision Pro Ltd., Wuxi, Jiangsu 214125 (CN)
(72) Inventor: GUO, Feng, Wuxi, Jiangsu 214125 (CN); ZHOU, Mengdie, Wuxi, Jiangsu 214125 (CN); LIAO, Xiugao, Wuxi, Jiangsu 214125 (CN); FENG, Zhenyu, Wuxi, Jiangsu 214125 (CN)
(74) Representative: Petraz, Gilberto Luigi
(86) International application number: PCT/CN2024/078485
(87) International publication number: WO 2025/166851

(57) **Abstract**

The present utility model discloses a pre-loaded intraocular lens injection system, including: a main cylinder, a plunger, an advancing knob, and a cartridge, where an introduction channel is formed inside the cartridge; a mounting recess for containing an intraocular lens is formed in the introduction channel, and a detachable lens limiting member is disposed on the mounting recess; and the lens limiting member includes a limiting plate, a stop block for preventing the plunger from extending into the introduction channel is disposed on the limiting plate in a movement direction of the plunger, and an anti-straight-haptic column is disposed on the limiting plate on a side of the movement direction of the plunger. According to the pre-loaded intraocular lens injection system, rotation and pushing functions are integrated, so that the injection accuracy is high; the limiting plate is configured to prevent the lens from being overturned, and the stop block and the anti-straight-haptic column are configured to prevent a front haptic from coming out straight, so that when being injected, the intraocular lens maintains such a posture that two haptics hold and cover an optical region; meanwhile, deformation of the intraocular lens can be avoided, and the stable position of the intraocular lens is ensured; and the system is easy and efficient to operate.

## Description

### TECHNICAL FIELD

The present utility model belongs to the technical field of auxiliary medical instruments, and particularly relates to a pre-loaded intraocular lens injection system.

### BACKGROUND

An intraocular lens is a medical instrument that can be injected into an eye of a patient to replace a diseased natural lens, is currently the most effective and direct means for correcting aphakia ocular refraction, and needs to be pushed into the eye by means of an injection system. An existing pre-loaded intraocular lens injection system is composed of the intraocular lens and an injection device, and the product is applicable to eye lens replacement in an operation for an ophthalmic disease such as cataracts. When an intraocular lens injection operation is performed, the pre-loaded intraocular lens injection system combines the loaded intraocular lens and the injection device for use, compared with a non-pre-loaded system, the system has the advantages that the intraocular lens is not required to be clamped by a forceps, surgical efficiency would be improved, the infection possibility of the patient would be reduced, and thus the system is widely applied to the intraocular lens injection operation.

When the injecting or rotating type pre-loaded intraocular lens injection system is used to inject the intraocular lens, a component for containing the intraocular lens and a pushing component of the intraocular lens injection system need to be combined, and the intraocular lens is pushed into the eye by means of the pushing component. However, due to the intraocular lens itself is soft, there is a risk of movements in its position during transportation, which may require manual correction of the lens position and cause inconvenience; meanwhile, when the intraocular lens is pushed by the pushing component, it often occurs that the lens flips and the front haptic is straight out, so that the intraocular lens cannot be injected into the eye in such a posture that the two haptics hold and cover an optical region of the lens, resulting in the problems of large injection cut of the eye and unsuccessful operation.

Prior art, the Chinese utility model patent with the publication number of CN212662028U, discloses an intraocular lens injector, where a main cylinder can be mounted in cooperation with an intraocular lens storage box preloaded with an intraocular lens, and a box cavity of the intraocular lens storage box is located inside the main cylinder and forms a box cavity opening at the bottom of the main cylinder; and a pressing block opening is formed in the position directly above the box cavity opening in the main cylinder, and a pressing block turned downwards enters the box cavity through the pressing block opening, then comes into contact with the intraocular lens and presses the intraocular lens into a state in which the middle thereof is recessed. The pressing block can fix the lens to a certain extent; however, the lens is a soft elastic material component, so that it is prone to damage under the simultaneous compression and injection action, and the phenomenon of straight coming-out of the front haptic cannot be avoided. On this basis, it is necessary to develop a pre-loaded intraocular lens injection system, which can maintain two haptics holding and covering an optical region of the lens during injection of the intraocular lens without the risk of damaging the intraocular lens, and the lens is not-prone to overturning, thereby ensuring the safety and stability of the lens injection operation.

### SUMMARY

In order to overcome the above defects in the prior art, the present utility model provides a pre-loaded intraocular lens injection system. The pre-loaded intraocular lens injection system can maintain two haptics holding and covering an optical region of a lens during injection of the intraocular lens without the risk of damaging the intraocular lens, and the lens is not prone to overturning, thereby ensuring the safety and stability of a lens injection operation and the transportation stability of the pre-loaded intraocular lens injection system.

In order to achieve the above purposes of the utility model, the technical solution adopted in present utility model is as follows:
a pre-loaded intraocular lens injection system, including:
a main cylinder, where the main cylinder includes a cavity with openings formed at two ends of the main cylinder;
a plunger, where one end of the plunger extends into the cavity along the opening at one end of the main cylinder and extends along the cavity towards the opening at the other end of the main cylinder;
an advancing knob, where the advancing knob is disposed between the main cylinder and the plunger, and the plunger is driven by the advancing knob to ascend and descend relative to the cavity;
a cartridge, where the cartridge is disposed at the opening of the end of the main cylinder away from the plunger, and an introduction channel is formed inside the cartridge; a mounting recess for containing an intraocular lens is formed on one side of the introduction channel close to the opening of the main cylinder, and a detachable lens limiting member is disposed on the mounting recess; the lens limiting member includes a limiting plate, a stop block for preventing the plunger from extending into the introduction channel is disposed on the limiting plate on a rear side of a movement direction of the plunger, and an anti-straight-haptic column is disposed on the limiting plate on a front side of the movement direction of the plunger;
one end of the plunger extends into the cavity along the advancing knob and enters an inlet of the introduction channel of the cartridge along the cavity to abut against the intraocular lens located inside the cartridge, so as to inject the intraocular lens into an eye along the introduction channel.

The present utility model relates to the rotation and pushing integrated pre-loaded intraocular lens injection system, the advancing knob is matched with the plunger for use, the plunger can be directly pushed to push the intraocular lens to a preparation position, then the advancing knob is rotated, the plunger is then slowly pushed further, and the intraocular lens is accurately pushed out of the cartridge; the detachable lens limiting member is disposed on the mounting recess of the introduction channel; the lens limiting member includes the limiting plate for preventing the lens from being overturned, and the limiting plate is provided with the stop block for preventing the plunger from extending into the introduction channel and the anti-straight-haptic column on the front side of the movement direction of the plunger for preventing the front haptic from coming out straight; during the injection of the intraocular lens, it can not only prevent excessive injection force from causing eye damage, but also ensure that the intraocular lens maintains the posture of two haptics holding and covering the optical region when injected into the eye; and the stop block can prevent the displacement of the plunger during transportation, thereby ensuring the position stability of the lens, and ensuring that an operator can safely and stably inject the intraocular lens into the eye.

Further, the end of the plunger extending towards the opening at the other end of the main cylinder is of a groove-shaped structure capable of wrapping a backside haptic of the intraocular lens.

Further, a clamping device for fixing the lens limiting member to the mounting recess or detaching the lens limiting member from the mounting recess is disposed on the limiting plate.

Further, the clamping device includes a clamping portion and a detachment handle, and the clamping portion and the detachment handle are hinged to the limiting plate.

Further, an injection orifice is disposed on the limiting plate.

Further, the advancing knob is a sleeve with openings at two ends, an outer wall surface of the plunger is in threaded connection with the sleeve, and the plunger passes through the sleeve to enter the cavity of the main cylinder.

Further, at least one protrusion is disposed on an inner circumferential wall of one end of the sleeve away from the cartridge, a threaded tooth is disposed on the at least one protrusion, and a threaded groove fitted with the threaded tooth is formed in the outer wall surface of the plunger.

Further, an inner wall surface of the sleeve is rotationally connected to an outer wall surface of the main cylinder.

Further, the main cylinder is rotationally connected to the advancing knob by means of limiting components; and the limiting components include a limiting groove and at least one limiting portion, the limiting groove is formed in an outer circumferential surface of the main cylinder, any limiting portion is disposed on an inner circumferential surface of the advancing knob, after the advancing knob is mounted on the main cylinder, the limiting portion is located inside the limiting groove, and the limiting portion is capable of rotating inside the limiting groove along the outer circumferential surface of the main cylinder, so as to enable the advancing knob to rotate relative to the main cylinder.

Preferably, the groove depth of the limiting groove is greater than the height of any limiting portion, and/or the groove length of the limiting groove is equal to the axial length of any limiting portion, thereby preventing the limiting portion from moving axially relative to an axial direction of the main cylinder.

Preferably, the limiting portion is configured as a wedge block.

Further, the plunger is further sleeved with a sealing ring, and the sealing ring hermetically abuts against an inner wall surface of the cavity.

Preferably, a sealing groove for mounting the sealing ring is formed in the plunger.

The beneficial effects of the present utility model are as follows:
(1) according to the pre-loaded intraocular lens injection system provided in the present utility model, rotation and pushing functions are integrated, so that the intraocular lens located inside the introduction channel of the cartridge can be accurately and safety injected into the eye, and the damage to the eye is avoided to the maximum extent;
(2) according to the pre-loaded intraocular lens injection system provided in the present utility model, the front haptic can be prevented from coming out straight when the intraocular lens is pushed out, meanwhile, the displacement of the plunger during transportation is limited, the deformation of the lens is avoided, and the position stability of the lens is ensured; in addition, the system is easy and efficient to operate, and the use feeling of a doctor during an operation and the operation safety of a patient are all improved.

### BRIEF DESCRIPTION OF DRAWINGS

FIG. 1 is an overall structural schematic diagram of an injection system in the present utility model.
FIG. 2 is a structural schematic diagram of a lens limiting member in the embodiment.
FIG. 3 is a schematic diagram of a connecting structure of an advancing knob and a main cylinder as shown in FIG. 1.
FIG. 4 is a structural schematic diagram of the advancing knob.
FIG. 5 is a top-view structural schematic diagram of the advancing knob.
FIG. 6 is a sectional structural schematic diagram of the advancing knob as shown in FIG. 3.
FIG. 7 is a structural schematic diagram of an plunger in the embodiment.

The description of reference numerals: 1. main cylinder; 2. plunger; 3. advancing knob; 4. cartridge; 5. protrusion; 6. threaded tooth; 7. threaded groove; 8. limiting groove; 9. limiting portion; 10. cavity; 11. sealing ring; 12. sealing groove; 13. mounting recess; 14. lens limiting member; 15. limiting plate; 16. detachment handle; 17. anti-straight-haptic column; 18. injection orifice; 19. stop block; and 20. clamping portion.

### DETAILED DESCRIPTION OF EMBODIMENTS

Specific embodiments of the present utility model are further described below in conjunction with the accompanying drawings. The same parts are represented by the same reference numerals. It should be noted that the words "front", "rear", "left", "right", "upper" and "lower" used in the following description refer to directions in the accompanying drawings, and the words "inner" and "outer" respectively refer to directions towards or away from the geometric center of a specific part. In order to understand the content of the present utility model more easily and clearly, the technical solutions in the embodiments of the present utility model will be described clearly and completely below in conjunction with the accompanying drawings in the embodiments of the present utility model.

### Embodiment 1

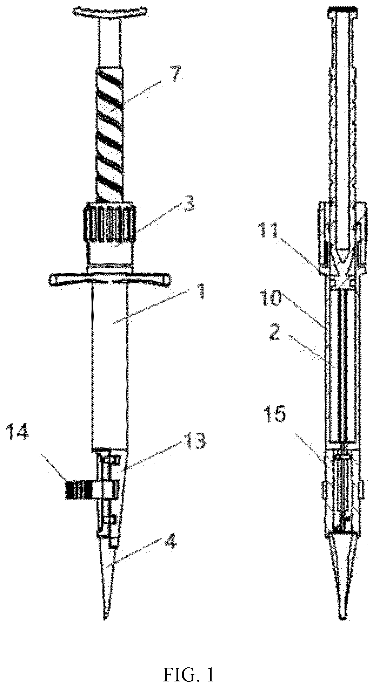

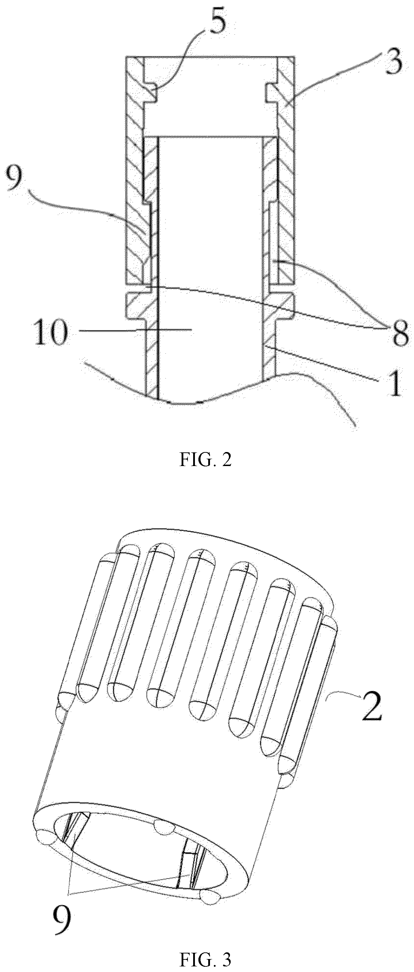

A pre-loaded intraocular lens injection system, as shown in FIG. 1 and FIG. 2, includes: a main cylinder 1, where the main cylinder 1 includes a cavity 10 with openings formed at two ends; an plunger 2, where one end of the plunger 2 extends into the cavity 10 along the opening at one end of the main cylinder 1 and extends along the cavity 10 towards the opening at the other end of the main cylinder 1; an advancing knob 3, where the advancing knob 3 is disposed between the main cylinder 1 and the plunger 2, and the plunger 2 is driven by the advancing knob 3 to ascend and descend relative to the cavity 10; and a cartridge 4, where the cartridge 4 is disposed at the opening of the end of the main cylinder 1 away from the plunger 2, and an introduction channel is formed inside the cartridge 4; a mounting recess 13 for containing an intraocular lens is formed on one side of the introduction channel close to the opening of the main cylinder 1, and a detachable lens limiting member 14 is disposed on an upper portion of the mounting recess 13; the lens limiting member 14 includes a limiting plate 15, a stop block 19 for preventing the plunger 2 from extending into the introduction channel is disposed on the limiting plate 15 on a rear side of a movement direction of the plunger 2, and an anti-straight-haptic column 17 is disposed on the limiting plate 15 on a front side of the movement direction of the plunger 2. One end of the plunger 2 extends into the cavity 10 along the advancing knob 3, and stops when being subjected to considerable resistance. Then, a detachment handle 16 is pinched, the lens limiting member 14 is pulled out vertically upwards, and the end of the plunger 2 continuously enters an inlet of the introduction channel of the cartridge 4 along the cavity 10 to abut against the intraocular lens located inside the introduction channel, so as to inject the intraocular lens into an eye along the introduction channel.

In a specific implementation process, according to the pre-loaded intraocular lens injection system, when the intraocular lens needs to be injected, the plunger 2 is pushed firstly, and a backside haptic of the intraocular lens is pushed to be bent in place; the lens limiting member 14 is disposed on the mounting recess 13 of the introduction channel; the lens limiting member 14 includes the limiting plate 15, and the limiting plate 15 and the mounting recess 13 limit the intraocular lens vertically to prevent the intraocular lens from being overturned; meanwhile, the anti-straight-haptic column 17 on the side of the movement direction of the plunger 2 is disposed on the limiting plate 15 and is located on a front side of a front haptic of the intraocular lens, and thus the intraocular lens moves along the introduction channel in such a posture that the two haptics hold and cover an optical region so that the front haptic can be prevented from coming out straight; the stop block 19 is disposed in the movement direction of the plunger 2, and the stop block 19 can prevent further movement of the plunger 2 in the introduction channel and together with the anti-straight-haptic column 17 prevent the front haptic from coming out straight; and when the intraocular lens is injected, the lens limiting member 14 is removed, the plunger 2 is slowly pushed by means of the advancing knob 3 to safely, stably and accurately push the intraocular lens into the eye, so that damage to the eye due to the excessive injection force can be prevented. Meanwhile, the stop block 19 can prevent displacement of the plunger 2 during transportation, thereby ensuring the position stability of the intraocular lens.

According to the pre-loaded intraocular lens injection system provided in the present utility model, rotation and pushing functions are integrated, so that the intraocular lens located inside the introduction channel of the cartridge 4 can be accurately and safety injected into the eye, and the damage to the eye is avoided to the maximum extent; according to the injection system, the front haptic can be prevented from coming out straight when the intraocular lens is pushed out, meanwhile, the displacement of the plunger 2 during transportation is limited, the deformation of the lens is avoided, and the position stability of the lens is ensured. In addition, the system is easy and efficient to operate, and the use feeling of a doctor during an operation and the operation safety of a patient are improved.

### Embodiment 2

To further achieve the integration of the rotation and pushing functions of the pre-loaded intraocular lens injection system, on the basis of Embodiment 1, as an optional solution, the advancing knob 3 is a sleeve with openings at two ends, an outer wall surface of the plunger 2 is in threaded connection with the sleeve, and the plunger 2 passes through the sleeve to enter the cavity 10 of the main cylinder 1.

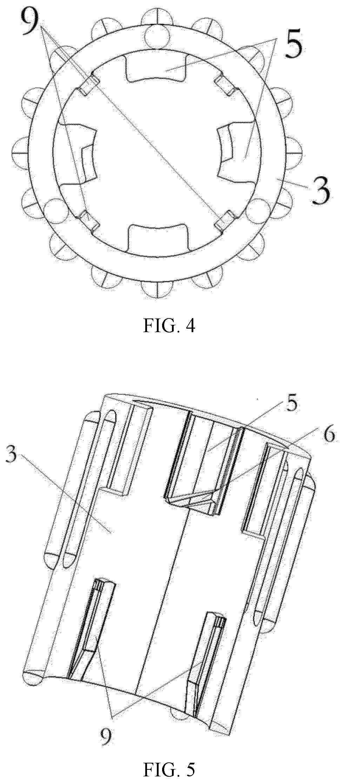

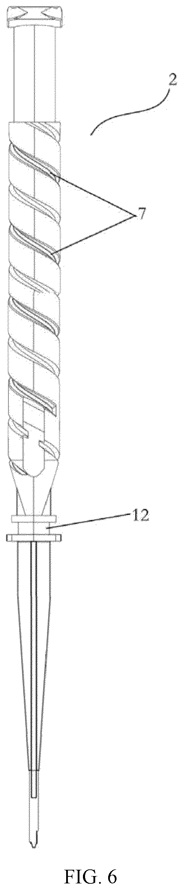

Reference is made to FIGS. 3-6, and as an optional solution, at least one protrusion 5 is disposed on an inner circumferential wall of one end of the sleeve away from the cartridge 4, a threaded tooth 6 is disposed on the at least one protrusion 5, and a threaded groove 7 fitted with the threaded tooth 6 is formed in the outer wall surface of the plunger 2; and according to actual requirements, two or four protrusions 5 may be provided, and each protrusion 5 is provided with the threaded tooth 6. As an optional solution, an inner wall surface of the sleeve is rotationally connected to an outer wall surface of the main cylinder 1.

As an optional solution, the main cylinder 1 is rotationally connected to the advancing knob 3 by means of limiting components; and the limiting components include a limiting groove 8 and at least one limiting portion 9, the limiting groove 8 is formed in an outer circumferential surface of the main cylinder 1, any limiting portion 9 is disposed on an inner circumferential surface of the advancing knob 3, after the advancing knob 3 is mounted on the main cylinder 1, the limiting portion 9 is located inside the limiting groove 8, and the limiting portion 9 is capable of rotating inside the limiting groove 8 along the outer circumferential surface of the main cylinder 1, so as to enable the advancing knob 3 to rotate relative to the main cylinder 1. Preferably, the groove depth of the limiting groove 8 is greater than the height of any limiting portion 9. As an optional solution, the groove length of the limiting groove 8 is equal to the axial length of any limiting portion 9, thereby preventing the limiting portion 9 from moving axially relative to an axial direction of the main cylinder 1. Preferably, the limiting portion 9 is arranged as a wedge block. There may be one limiting portion 9, and of course, there may also be two or four limiting portions.

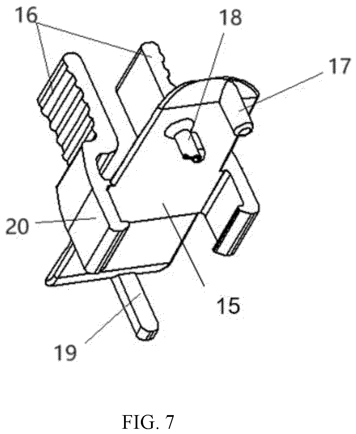

To improve the sealing performance of the injection system, reference is made to FIG. 7, as an optional solution, the plunger 2 is further sleeved with a sealing ring 11, and the sealing ring 11 hermetically abuts against an inner wall surface of the cavity 10; and preferably, the sealing ring 11 is an O-ring. Preferably, a sealing groove 12 for mounting the sealing ring 11 is formed in the plunger 2.

In a specific implementation process, when the rotating block 3 is operated to rotate relative to the outer circumferential surface of the main cylinder 1, the sleeve is in threaded connection with the plunger 2, the torque of the sleeve can be converted into an axial repeated action force which acts on the plunger 2, thus the plunger 2 can axially ascend and descend along the cavity 10 of the main cylinder 1, and when the plunger 2 descends in the axial direction of the cavity 10, the intraocular lens can be pushed out of the cartridge 4.

When the rotating block 3 needs to rotate, the limiting portion 9 can rotate in the limiting groove 8 along the outer circumferential surface of the main cylinder 1, so that the rotating block 3 rotates relative to the main cylinder 1; in order to prevent the rotating block 3 from being separated from the limiting groove 8, the groove depth of the limiting groove 8 can be greater than the height of any limiting portion 9, then when the rotating block 3 rotates relative to the main cylinder 1, it cannot be separated from the limiting groove 8; and of course, the groove length of the limiting groove 8 can also be set to be equal to the axial length of any limiting portion 9, so that two end wall surfaces of the limiting portion 9 abut against two end wall surfaces of the limiting groove 8 which are in contact with the limiting portion 9, and can rotate along the limiting groove 8. Then, the limiting portion 9 can be prevented from axially moving relative to the axial direction of the main cylinder 1, the limiting portion 9 cannot be separated from the limiting groove 8, the torque of the sleeve can be converted into the axial repeated action force on the plunger 2, the intraocular lens can be slowly and accurately injected, and the injection damage to the eye is reduced.

### Embodiment 3

To maintain the posture that the two haptics hold and cover the optical region and to prevent the front haptic from coming out straight when the intraocular lens moves along the introduction channel, on the basis of Embodiment 1 or Embodiment 2, as an optional solution, the end of the plunger 2 extending towards the opening at the other end of the main cylinder 1 is of a groove-shaped structure capable of wrapping a backside haptic of the intraocular lens. Preferably, the groove is a U-shaped groove.

In a specific implementation process, when the U-shaped groove at the end of the plunger 2 extending towards the opening at the other end of the main cylinder 1 moves towards the intraocular lens, the backside haptic of the intraocular lens can hold and cover the optical region.

### Embodiment 4

To facilitate detachment and use of the lens limiting member 14, on the basis of Embodiment 1 or Embodiment 2, reference is made to FIG. 2, as an optional solution, a clamping device for fixing the lens limiting member 14 to the mounting recess 13 or detaching the lens limiting member from the mounting recess 13 is disposed on the limiting plate 15. Preferably, the clamping device includes a clamping portion 20 and a detachment handle 16, and the clamping portion 20 and the detachment handle 16 are hinged to the limiting plate 15.

As an optional solution, an injection orifice 18 is disposed on the limiting plate 15, and the injection orifice 18 is configured to supplement or add a liquid for protecting or lubricating the intraocular lens; and preferably, the liquid is sodium hyaluronate gel.

In a specific implementation process, when the clamping device needs to be fixed or detached, all that is needed is to press the detachment handle 16, and then the clamping portion 20 is fixed to the mounting recess 13 or is detached from the mounting recess 13.

The above are only the preferred embodiments of the present utility model patent, and do not constitute limitations on the present utility model patent. Any modification, equivalent replacement and improvement made within the spirit and principles of the present utility model patent should fall within the scope of protection of the present utility model patent.

## Claims

1. A pre-loaded intraocular lens injection system, **characterized in that**, the system comprises:
a main cylinder (1), wherein the main cylinder (1) comprises a cavity (10) with openings formed at two ends;
an plunger (2), wherein one end of the plunger (2) extends into the cavity (10) from the opening formed at one end of the main cylinder (1) and extends along the cavity (10) towards the opening formed at the other end of the main cylinder (1);
an advancing knob (3), wherein the advancing knob (3) is disposed between the main cylinder (1) and the plunger (2), and the plunger (2) is driven by the advancing knob (3) to ascend and descend relative to the cavity (10); and
a cartridge (4), wherein the cartridge (4) is disposed at the opening of the end of the main cylinder (1) away from the plunger (2), and an introduction channel is formed inside the cartridge (4); a mounting recess (13) for containing an intraocular lens is formed on one side of the introduction channel close to the opening of the main cylinder (1), and a detachable lens limiting member (14) is disposed on the mounting recess (13);
the lens limiting member (14) comprises a limiting plate (15), a stop block (19) for preventing the plunger (2) from extending into the introduction channel is disposed on the limiting plate (15) in a movement direction of the plunger (2), and an anti-straight-haptic column (17) is disposed on the limiting plate (15) on a side of the movement direction of the plunger (2); and
one end of the plunger (2) extends into the cavity (10) along the advancing knob (3) and enters an inlet of the introduction channel of the cartridge (4) along the cavity (10) to abut against the intraocular lens located inside the introduction channel, so as to inject the intraocular lens along the introduction channel into an eye.

2. The pre-loaded intraocular lens injection system according to claim 1, wherein an other end of the plunger (2) extending towards the opening formed at the other end of the main cylinder (1) is of a groove-shaped structure capable of wrapping a backside haptic of the intraocular lens.

3. The pre-loaded intraocular lens injection system according to claim 1, wherein a clamping device for fixing the lens limiting member (14) to the mounting recess (13) or detaching the lens limiting member from the mounting recess (13) is disposed on the limiting plate (15); and
preferably, the clamping device comprises a clamping portion (20) and a detachment handle (16), and the clamping portion (20) and the detachment handle (16) are hinged to the limiting plate (15).

4. The pre-loaded intraocular lens injection system according to claim 1, wherein an injection orifice (18) is disposed on the limiting plate (15).

5. The pre-loaded intraocular lens injection system according to claim 1, wherein the advancing knob (3) is a sleeve with openings at two ends, an outer wall surface of the plunger (2) is in threaded connection with the sleeve, and the plunger (2) passes through the sleeve to enter the cavity (10) of the main cylinder (1).

6. The pre-loaded intraocular lens injection system according to claim 5, wherein at least one protrusion (5) is disposed on an inner circumferential wall of one end of the sleeve away from the cartridge (4), a threaded tooth (6) is disposed on the at least one protrusion (5), and a threaded groove (7) fitted with the threaded tooth (6) is formed in the outer wall surface of the plunger (2).

7. The pre-loaded intraocular lens injection system according to claim 5, wherein an inner wall surface of the sleeve is rotationally connected to an outer wall surface of the main cylinder (1).

8. The pre-loaded intraocular lens injection system according to claim 5, wherein the main cylinder (1) is rotationally connected to the advancing knob (3) by means of limiting components; and the limiting components comprise a limiting groove (8) and at least one limiting portion (9), the limiting groove (8) is formed in an outer circumferential surface of the main cylinder (1), any of the at least one limiting portion (9) is disposed on an inner circumferential surface of the advancing knob (3), after the advancing knob (3) is mounted on the main cylinder (1), the limiting portion (9) is located inside the limiting groove (8), and the limiting portion (9) is capable of rotating inside the limiting groove (8) along the outer circumferential surface of the main cylinder (1), so as to enable the advancing knob (3) to rotate relative to the main cylinder (1).

9. The pre-loaded intraocular lens injection system according to claim 8, wherein a groove depth of the limiting groove (8) is greater than a height of any of the at least one limiting portion (9), and/or a groove length of the limiting groove (8) is equal to an axial length of any of the at least one limiting portion (9), thereby preventing the limiting portion (9) from moving axially relative to an axial direction of the main cylinder (1).

10. The pre-loaded intraocular lens injection system according to claim 9, wherein the plunger (2) is further sleeved with a sealing ring (11), and the sealing ring (11) hermetically abuts against an inner wall surface of the cavity (10).
